# EUROPEAN PATENT APPLICATION

(11) **EP 4 620 452 A1**
(43) Date of publication of application: **24.09.2025**
(21) Application number: 23891737.1
(22) Date of filing: 19.06.2023
(51) Int. Cl.: A61K 8/04, A61K 8/73, A61K 8/34, A61Q 19/00

(54) **ION-CURABLE HYDROGEL COMPOSITION COMPRISING EXTRACELLULAR POLYSACCHARIDES**

(30) Priority: 15.11.2022 KR 20220152317
(71) Applicant: Kolmar Korea Co., Ltd., Sejong 30004 (KR)
(72) Inventor: JEONG, Eun Sook, Seoul 06800 (KR); BAE, Hyung Jin, Seoul 06800 (KR); KIM, Jin Mo, Seoul 06800 (KR)
(74) Representative: Gulde & Partner
(86) International application number: PCT/KR2023/008442
(87) International publication number: WO 2024/106662

(57) **Abstract**

An embodiment of the present invention provides a hydrogel composition. The hydrogel composition comprises: a first gelling agent; and a second gelling agent, wherein the first gelling agent comprises at least one of an alginate, a carrageenan, and a gellan gum, and the second gelling agent can be a succinoglycan.

## Description

### [Technical Field]

The present invention relates to an ion-curable hydrogel composition including extracellular polysaccharides, and more particularly, to a hydrogel composition which includes succinoglycan as a second gelling agent in addition to a first gelling agent, thereby preventing syneresis and providing an excellent feeling of use.

### [Background Art]

Hydrogels are materials in which hydrophilic polymer chains are cross-linked to form a three-dimensional network structure. They contain a large amount of water inside and have unique properties such as being thermodynamically stable in aqueous solutions so that they are actively used in various fields such as medicine, beauty care, and industry.

These hydrogels contain gelling agents such as carrageenan, cellulose, and xanthan gum to form a solid film. However, conventional hydrogels manufactured using only these gelling agents have problems such as syneresis, which is water separation from the gel, and a heavy feeling of use.

### [Disclosure]

### [Technical Problem]

The present invention is intended to solve the above-described problems, and an object of the present invention is to provide a hydrogel composition that contains a large amount of water while minimizing syneresis and providing excellent feelings of skin application and use.

The technical problems of the present invention are not limited to the above-mentioned technical problems, and other technical problems that are not mentioned will be clearly understood by those skilled in the art from the descriptions below.

### [Technical Solution]

According to an embodiment of the present invention, there is provided a hydrogel composition. The hydrogel composition may include: a first gelling agent; and a second gelling agent, and the first gelling agent may include alginate, carrageenan, and gellan gum, and the second gelling agent may be succinoglycan.

In addition, the alginate, the carrageenan, the gellan gum, and the succinoglycan may be included in a weight ratio of 1:0.5 to 8:0.5 to 2:0.5 to 4.

In addition, the alginate may be included in an amount of 0.01% to 10% by weight based on the total weight of the composition.

In addition, the carrageenan may be included in an amount of 0.01% to 10% by weight based on the total weight of the composition.

In addition, the gellan gum may be included in an amount of 0.01% to 10% by weight based on the total weight of the composition.

In addition, the succinoglycan may be included in an amount of 0.01% to 10% by weight based on the total weight of the composition.

In addition, the first gelling agent may further include one or more selected from the group consisting of glucomannan, xanthan gum, gelatin, chitosan, collagen, hyaluronic acid, gum arabic tree (*Acacia senegal* gum), pullulan, pectin, cellulose, sclerotium gum, tara gum (*Caesalpinia spinosa* gum), agar, locust bean gum, and guar gum (*Cyamopsis tetragonoloba* gum).

In addition, the composition may further include at least one of a curing agent and a moisturizing agent.

In addition, the curing agent may include at least one of a divalent metal ion, a trivalent metal ion, and a metal salt.

In addition, the metal ion may include one or more selected from the group consisting of calcium (Ca²⁺), magnesium (Mg²⁺), barium (Ba²⁺), manganese (Mn²⁺), nickel (Ni²⁺), copper (Cu²⁺), zinc (Zn²⁺), iron (Fe³⁺), and aluminum (Al³⁺).

In addition, the metal salt may include one or more selected from the group consisting of calcium chloride, calcium hydroxide, calcium nitrate, and calcium sulfide.

In addition, the curing agent may be included in an amount of 0.01% to 5% by weight based on the total weight of the composition.

In addition, the moisturizing agent may include one or more selected from the group consisting of glycerin, propylene glycol, dipropylene glycol, butylene glycol, pentylene glycol, and sorbitol.

In addition, the moisturizing agent may be included in an amount of 0.01% to 35% by weight based on the total weight of the composition

According to an embodiment of the present invention, there is provided a method of preparing a hydrogel composition. The method may include: a step of mixing a first gelling agent and a second gelling agent in purified water to obtain a mixture; and a step of dropping the mixture into a curing agent, and the first gelling agent may include alginate, carrageenan, and gellan gum, and the second gelling agent may be succinoglycan.

### [Advantageous Effects]

The hydrogel composition according to the present invention has excellent physical properties such as swelling, wettability, and strength and has the characteristic of containing a large amount of water inside a gel while minimizing water evaporation and syneresis.

In addition, the hydrogel composition according to the present invention has the advantage that it can be easily applied to the skin and provide an excellent rolling sensation and residual sensation.

### [Modes of the Invention]

Unless otherwise defined, all technical and scientific terms used herein have the same meaning as commonly understood by one of ordinary skill in the art to which the present invention pertains. In general, the nomenclature used herein is well known and commonly used in the art. In addition, when describing embodiments of the present invention, when a detailed description of a related known feature or function is judged to hinder the understanding of the embodiments of the present invention, the detailed description thereof will be omitted. In addition, although embodiments of the present invention will be described below, the technical idea of the present invention is not limited or restricted thereto and may be modified and implemented in various ways by those skilled in the art.

The present invention relates to a hydrogel composition, and more specifically, to a hydrogel composition having excellent properties such as swelling, wettability, and strength and providing an excellent feeling of use such as a rolling sensation or residual sensation.

The present inventors optimally adjusted hydrogel properties such as wettability, swelling, and strength by using an extracellular polysaccharide produced from a microorganism as a gelling agent, thereby developing a hydrogel composition having an excellent rolling sensation and residual sensation.

The hydrogel composition of the present invention may include two or more gelling agents.

The gelling agent may form a film to contain a large amount of water inside and may serve to adjust the strength of a hydrogel. Specifically, the gelling agent may include two gelling agents consisting of a first gelling agent and a second gelling agent. When the second gelling agent is included in addition to the first gelling agent, the strength of the gel is more stably adjusted, and the water content of the gel is increased, thereby providing an excellent moisturizing effect and feeling of use.

According to an embodiment, the first gelling agent may include at least one of alginate, carrageenan, and gellan gum. For example, the first gelling agent may include all of alginate, carrageenan, and gellan gum. When alginate, carrageenan, and gellan gum are mixed and the resulting mixture is used as the first gelling agent, the hydrogel can efficiently absorb water, and evaporation of the absorbed water can be minimized.

For example, alginate may be included in an amount of 0.01% to 10% by weight, specifically 0.01% to 5% by weight, and more specifically 0.01% to 1.2% by weight based on the total weight of the composition.

In addition, for example, carrageenan may be included in an amount of 0.01% to 10% by weight, specifically 0.01% to 5% by weight, and more specifically 0.01% to 2% by weight, based on the total weight of the composition.

As another example, gellan gum may be included in an amount of 0.01% to 10% by weight, specifically 0.01% to 5% by weight, and more specifically 0.01% to 1.2% by weight based on the total weight of the composition.

When alginate, carrageenan, or gellan gum is each included outside the above-mentioned content range, a gel may not be formed or, even when a gel is formed, the feeling of use may deteriorate.

According to an embodiment, the first gelling agent may further include one or more selected from the group consisting of glucomannan, xanthan gum, gelatin, chitosan, collagen, hyaluronic acid, gum arabic tree (*Acacia senegal* gum), pullulan, pectin, cellulose, sclerotium gum, tara gum (*Caesalpinia spinosa* gum), agar, locust bean gum, and guar gum (*Cyamopsis tetragonoloba* gum), but is not limited thereto.

According to an embodiment, the second gelling agent may be succinoglycan as an extracellular polysaccharide. Succinoglycan is a polysaccharide produced from a fermentation product of *Sinorhizobium meliloti* or *Agrobacterium tumefaciens,* and together with the first gelling agent, it may improve the strength of a hydrogel to prevent the gel from collapsing and enable the hydrogel to contain a greater amount of water while minimizing the evaporation of water from the hydrogel.

In general, existing hydrogel compositions include only a first gelling agent to form a hard film, and such hydrogel compositions have problems in that syneresis, which is water separation from the gel, occurs, or application and rolling on the skin are not easy, resulting in a poor feeling of use. However, the present invention includes succinoglycan as a second gelling agent in addition to a first gelling agent generally used as a gelling agent of hydrogels, thereby minimizing syneresis and providing excellent feelings of use, such as a smooth application sensation on the skin with a rolling sensation and a continuous moisturizing effect with a feeling of the composition remaining on the skin.

According to an embodiment, succinoglycan may be included in an amount of 0.01% to 10% by weight, specifically 0.01% to 5% by weight, and more specifically 0.01% to 1.2% by weight based on the total weight of the composition. When succinoglycan is included in an amount of less than 0.01% by weight based on the total weight of the composition, syneresis of a hydrogel may occur, and when succinoglycan is included in an amount of more than 10% by weight based on the total weight of the composition, the strength of the gel may become too high so that gel may collapse.

Specifically, in the present invention, for example, the first gelling agent may be alginate, carrageenan, and gellan gum, and the second gelling agent may be succinoglycan. The present inventors have confirmed that, when the first gelling agent and the second gelling agent are composed of the above-mentioned components, a synergistic effect is exhibited in terms of the physical properties (e.g., swelling, wettability, or strength) of the hydrogel and the feeling of use.

According to an embodiment, alginate, carrageenan, gellan gum, and succinoglycan may be included in a weight ratio of 1:0.5 to 8:0.5 to 2:0.5 to 4, and specifically, may be included in a weight ratio of 1:2 to 6:1 to 2:1 to 4. By adjusting the weight ratio of alginate, carrageenan, gellan gum, and succinoglycan within the above-mentioned range, a hydrogel film may be formed firmly so that the shape does not collapse, syneresis is minimized, and the feeling of use, such as the rolling sensation or residual sensation, is improved.

In this way, the present invention includes succinoglycan as a second gelling agent in addition to a first gelling agent generally used as a gelling agent of hydrogels, thereby stably adjusting the physical properties of hydrogels containing a large amount of water, such as strength, swelling, and wettability, and minimizing syneresis. In addition, the hydrogel according to the present invention provides an excellent feeling of use, such as the rolling sensation or residual sensation.

According to an embodiment, the composition may further include at least one of a curing agent and a moisturizing agent.

The curing agent may serve to adjust a gel curing rate. Specifically, the curing agent (e.g., metal ion) may form a crosslink with the first gelling agent and the second gelling agent as polysaccharides to form a hydrogel film.

According to an embodiment, the curing agent may include at least one of a divalent metal ion, a trivalent metal ion, and a metal salt. For example, the divalent metal ion may include one or more selected from the group consisting of calcium (Ca²⁺), magnesium (Mg²⁺), barium (Ba²⁺), manganese (Mn²⁺), nickel (Ni²⁺), copper (Cu²⁺), and zinc (Zn²⁺), and the trivalent metal ion may include one or more selected from the group consisting of iron (Fe³⁺) and aluminum (Al³⁺), but they are not limited thereto.

In addition, the metal salt may include one or more selected from the group consisting of calcium chloride, calcium hydroxide, calcium nitrate, and calcium sulfide, but is not limited thereto.

According to an embodiment, the curing agent may be included in an amount of 0.01% to 5% by weight based on the total weight of the composition. When the curing agent is included in an amount of less than 0.01% by weight based on the total weight of the composition, the curing time of the gel may be long, and when it is included in an amount of more than 5% by weight based on the total weight of the composition, curing may be too fast and thus formability may be reduced.

The moisturizing agent may serve to provide a moisturizing effect, such as supplying moisture to the skin or minimizing moisture evaporation.

According to an embodiment, the moisturizing agent may include one or more selected from the group consisting of glycerin, propylene glycol, dipropylene glycol, butylene glycol, pentylene glycol, and sorbitol, but is not limited thereto.

According to an embodiment, the moisturizing agent may be included in an amount of 0.01% to 35% by weight, and specifically, may be included in an amount of 0.01% to 30% by weight based on the total weight of the composition. When the moisturizing agent is included in an amount of less than 0.01% by weight based on the total weight of the composition, an insignificant moisturizing effect may be provided, and when it is included in an amount of more than 35% by weight based on the total weight of the composition, the strength or properties of the hydrogel may not be appropriately adjusted.

According to an embodiment, the hydrogel composition of the present invention may further include other components commonly used in the art, such as preservatives, thickeners, pH regulators, isotonic agents, surfactants, stabilizers, preservatives, ultraviolet absorbers, sterilizers, blocking agents, antioxidants, organic pigments, inorganic pigments, fragrances, vitamins, and the like within a range that does not impair the effects of the present invention. The mixing amount of the above-mentioned components may be easily selected by those skilled in the art within a range that does not impair the purpose and effects of the present invention.

According to an embodiment, the hydrogel composition of the present invention may be prepared as functional cosmetics such as nutritional creams, essence, or ampoules, or basic cosmetics such as toners or lotions. In addition, it may be prepared as soaps, detergents, cleansing creams, cleansing water, or the like, or hair cleansing products such as shampoos, rinses, hair conditioners, or hair gels. In addition, it may be prepared as products attached to the skin such as packs or hydrogel slimming patches, or it may be processed into a spherical shape and utilized in the form of capsules.

The hydrogel composition of the present invention may be prepared by the method described below.

First, a mixture may be obtained by mixing a first gelling agent and a second gelling agent in purified water. According to an embodiment, the first gelling agent may include at least one of alginate, carrageenan, and gellan gum. For example, the first gelling agent may include all of alginate, carrageenan, and gellan gum. When alginate, carrageenan, and gellan gum are mixed and used as the first gelling agent, a hydrogel can efficiently absorb water, and evaporation of the absorbed water can be minimized.

According to an embodiment, the second gelling agent may be succinoglycan. In the present invention, by mixing succinoglycan as a second gelling agent with purified water in addition to a first gelling agent generally used as a gelling agent of hydrogels, a composition that minimizes syneresis and provides excellent feelings of use, such as a feeling of being smoothly applied with a rolling sensation when applied to the skin and a feeling of continuously giving a moisturizing effect with a residual sensation of the composition remaining on the skin, may be prepared.

According to an embodiment, by appropriately adjusting the mixing ratio of the first gelling agent and the second gelling agent, the physical properties of the hydrogel and the feelings of use of the composition can be maximized. For example, when the first gelling agent is alginate, carrageenan, and gellan gum, and the second gelling agent is succinoglycan, the alginate, carrageenan, gellan gum, and succinoglycan may be mixed in a weight ratio of 1:0.5 to 8:0.5 to 2:0.5 to 4, and specifically, may be mixed in a weight ratio of 1:2 to 6:1 to 2:1 to 4.

According to the embodiment, in addition to the first gelling agent and the second gelling agent, other components commonly used in the art, such as preservatives, thickeners, pH regulators, isotonic agents, surfactants, stabilizers, preservatives, ultraviolet absorbers, sterilizers, blocking agents, antioxidants, organic pigments, inorganic pigments, fragrances, vitamins, and the like may be further mixed with purified water within a range that does not impair the effects of the present invention. The mixing amount of the above-mentioned components may be easily selected by those skilled in the art within a range that does not impair the purpose and effects of the present invention.

Thereafter, the mixture may be dropped into a curing agent. Accordingly, polymer chains of the mixture may be physically cross-linked so that the mixture is cured into a gel structure. Specifically, the first gelling agent and the second gelling agent as polysaccharides may form a cross-link with a divalent or higher metal ion such as Ca²⁺ ion to form a hydrogel film.

According to an embodiment, the curing agent may include at least one of a divalent metal ion, a trivalent metal ion, and a metal salt. For example, the divalent metal ion may include one or more selected from the group consisting of calcium (Ca²⁺), magnesium (Mg²⁺), barium (Ba²⁺), manganese (Mn²⁺), nickel (Ni²⁺), copper (Cu²⁺), and zinc (Zn²⁺), and the trivalent metal ion may include one or more selected from the group consisting of iron (Fe³⁺) and aluminum (Al³⁺), but they are not limited thereto.

In addition, for example, the metal salt may include one or more selected from the group consisting of calcium chloride, calcium hydroxide, calcium nitrate, and calcium sulfide, but is not limited thereto.

According to an embodiment, the dropping of the mixture may be performed by dropping the mixture in the form of drops into an aqueous solution containing a curing agent using a syringe pump. However, the mixture dropping method is not limited thereto, and various methods such as dropping using a dropper, a pipette, or a burette may be used.

According to an embodiment, as a method of forming the mixture into a gel structure, methods other than the dropping method may be applied. For example, a hydrogel composition may be prepared by pouring the mixture into a mold and molding the same. In this case, the hydrogel composition may be prepared into a hydrogel soap, a mask pack, a patch, and the like.

In addition, for example, a hydrogel composition may be prepared by a method of applying the mixture to a nonwoven fabric, attaching it to a release film, or prepared in the form of a patch through a series of processes such as rolling and cutting. In this case, the hydrogel composition may be prepared in the form of a mask pack, etc.

The hydrogel composition prepared in this manner includes a first gelling agent that is generally used as a gelling agent for hydrogels and further includes succinoglycan as a second gelling agent, so that the hydrogel film is formed firmly and syneresis is minimized. In addition, the hydrogel has excellent physical properties such as swelling, wettability, and strength and maximizes the feeling of use such as a rolling sensation or residual sensation.

### [Experimental Examples]

Hereinafter, examples are described to further explain the present invention, but the present invention is not limited thereto.

### Preparation of Examples and Comparative Examples

### (1) Preparation of Examples 1 to 5

Examples of the composition according to the present invention were prepared using the components described in Table 1 below. The unit of the content of the components in Table 1 is percentage by weight.

Examples 1 to 5 are hydrogel compositions where the weight ratio of the first gelling agent and the second gelling agent was adjusted within the scope of the examples of the present invention.

**[Table 1]**

| Role | | Component | Example 1 | Example 2 | Example 3 | Example 4 | Example 5 |
|---|---|---|---|---|---|---|---|
| Water | | | To 100 | To 100 | To 100 | To 100 | To 100 |
| Preservative | | 1,2-Hexanediol | 3 | 3 | 3 | 3 | 3 |
| Moisturizing agent | | Glycerin | 5 | 5 | 5 | 5 | 5 |
| Gelling agent | First gelling agent | Alginate | 0.25 | 0.2 | 0.25 | 0.2 | 0.5 |
| | | Carrageenan | 1 | 0.8 | 1 | 1.2 | 1 |
| | | Gellan gum | 0.25 | 0.2 | 0.5 | 0.2 | 0.5 |
| | Second gelling agent | Succinoglycan | 0.5 | 0.8 | 0.25 | 0.4 | 1 |
| | Total amount of gelling agents | | 2 | 2 | 2 | 2 | 3 |
| | Alginate: carrageenan: gellan gum: succinoglycan | | 1: 4 : 1 : 2 | 1 : 4 : 1 : 4 | 1 : 4 : 2 : 1 | 1 : 6 : 1 : 2 | 1:2: 1: 2 |
| Curing agent | Calcium chloride | 2 | 2 | 2 | 2 | 2 | |
| Total | | 100 | 100 | 100 | 100 | 100 | |

Specifically, a preservative, a moisturizing agent, and gelling agents were mixed in water and stirred uniformly. Thereafter, the resulting mixture was dropped into an aqueous solution containing a curing agent dissolved therein and cured to complete the compositions of Examples 1 to 5.

### (2) Preparation of Comparative Examples 1 to 11

Comparative examples of the composition according to the present invention were prepared using the components described in Table 2 below. The unit of the content of the components in Table 2 is percentage by weight.

Comparative Examples 1 to 11 are compositions where at least one of the four components constituting the first gelling agent and the second gelling agent is excluded.

Specifically, a preservative, a moisturizing agent, and gelling agents were mixed in water and stirred uniformly. Thereafter, the resulting mixture was dropped into an aqueous solution containing a curing agent dissolved therein and cured to complete the compositions of Examples 1 to 5.

### (2) Preparation of Comparative Examples 1 to 11

Comparative examples of the composition according to the present invention were prepared using the components described in Table 2 below. The unit of the content of the components in Table 2 is percentage by weight.

Comparative Examples 1 to 11 are compositions where at least one of the four components constituting the first gelling agent and the second gelling agent is excluded.

**[Table 2]**

| Role | | Component | Comparative Example 1 | Comparative Example 2 | Comparative Example 3 | Comparative Example 4 | Comparative Example 5 | Comparative Example 6 | ComparativeComparative Example 7 | Example 8 | Comparative Example 9 | Comparative Example 10 | Comparative Example 11 |
|---|---|---|---|---|---|---|---|---|---|---|---|---|---|
| Water | | | To 100 | To 100 | To 100 | To 100 | To 100 | To 100 | To 100 | To 100 | To 100 | To 100 | To 100 |
| Preservative | | Benzyl alcohol | 3 | 3 | 3 | 3 | 3 | 3 | 3 | 3 | 3 | 3 | 3 |
| Moisturizing agent | | Glycerin | 5 | 5 | 5 | 5 | 5 | 5 | 5 | 5 | 5 | 5 | 5 |
| Gelling agent | First gelling agent | Alginate | - | 1 | - | 1 | - | 1 | - | 1 | 1 | - | 1 |
| | | Carrageenan | 1 | - | - | - | 1 | 1 | - | 1 | 1 | 1 | - |
| | | Gellan gum | - | 1 | 1 | - | 1 | - | - | - | 1 | 1 | 1 |
| | Second gelling agent | Succinoglycan | 1 | - | 1 | 1 | - | - | 2 | 1 | - | 1 | 1 |
| | Total amount of gelling agents | | 2 | 2 | 2 | 2 | 2 | 2 | 2 | 3 | 3 | 3 | 3 |
| Curing agent I | | Calcium chloride | 2 | 2 | 2 | 2 | 2 | 2 | 2 | 2 | 2 | 2 | 2 |
| Total | | | 100 | 100 | 100 | 100 | 100 | 100 | 100 | 100 | 100 | 100 | 100 |

The specific preparation method is the same as in Examples 1 to 5.

### (2) Preparation of Comparative Examples 12 to 15

Comparative examples of the composition according to the present invention were prepared using the components described in Table 3 below. The unit of the content of the components in Table 2 is percentage by weight.

Comparative Examples 12 to 15 are compositions where the ratio of the first gelling agent and the second gelling agent is adjusted outside the scope of the examples of the present invention.

**[Table 3]**

| Role | Component | Comparative Example 1 | Comparative Example 2 | Comparative Example 3 | Comparative Example 4 | |
|---|---|---|---|---|---|---|
| Water | | To 100 | To 100 | To 100 | To 100 | |
| Preservative | Benzyl alcohol | 3 | 3 | 3 | 3 | |
| Moisturizing agent | Glycerin | 5 | 5 | 5 | 5 | |
| Gelling agent | First gelling agent | Alginate | 0.25 | 0.2 | 0.25 | 0.2 |
| | | Carrageenan | 1 | 0.8 | 1 | 1.2 |
| | | Gellan gum | 0.25 | 0.2 | 0.5 | 0.2 |
| | Second gelling agent | Succinoglycan | 0.5 | 0.8 | 0.25 | 0.4 |
| | Total amount of gelling agents | | 2 | 2 | 2 | 2 |
| | Alginate: carrageenan: gellan gum: succinoglycan | | 1: 4 : 1 : 2 | 1 : 4 : 1 : 4 | 1 : 4 : 2 : 1 | 1 : 6 : 1 : 2 |
| Curing agent | | Calcium chloride | 2 | 2 | 2 | 2 |
| Total | | | 100 | 100 | 100 | 100 |

The specific preparation method is the same as in Examples 1 to 5.

### Experimental Examples

### (1) Experimental Example 1: Evaluation of the properties of hydrogels

For the compositions according to Examples 1 to 5 and Comparative Examples 1 to 15, swelling, wettability, and strength were evaluated as the properties of the hydrogels, and the results are shown in Table 4.

The experimental method is as follows. For swelling, the samples of the examples and comparative examples were immersed in water with the same weight, and the degree of change in weight was measured to evaluate the swelling. The greater the weight after immersing in water, the more water the hydrogel absorbs, and thus the hydrogel is evaluated to have superior swelling.

The wettability was evaluated by exposing the samples of the examples and comparative examples to the outside for 24 hours and then measuring the degree of moisture evaporation. The lower the absolute value of the measured negative (-) value, the less evaporation of the moisture in the hydrogel, and thus the hydrogel is evaluated to have superior wettability.

The strength of the hydrogel was measured using a RHEOTECH FUDOH RHEO METER RTC-3005 D rheometer. When the strength is very high, exceeding a certain value, the hydrogel is easily broken. Therefore, when the strength has a value of 20 or less, the hydrogel may be evaluated to be hard and have excellent properties.

**[Table 4]**

| Classification | Swelling (%) | Wettability (%) | Strength (dyne/cm²) |
|---|---|---|---|
| Example 1 | 27 | -22 | 13 |
| Example 2 | 29 | -20 | 13 |
| Example 3 | 21 | -26 | 18 |
| Example 4 | 60 | -6 | 4 |
| Example 5 | 20 | -26 | 19 |
| Comparative Example 1 | 7 | -41 | 3 |
| Comparative Example 2 | 15 | -33 | 11 |
| Comparative Example 3 | -2 | -45 | 58 |
| Comparative Example 4 | 16 | -33 | 84 |
| Comparative Example 5 | 7 | -39 | 40 |
| Comparative Example 6 | 6 | -42 | 35 |
| Comparative Example 7 | 5 | -45 | 0 |
| Comparative Example 8 | -3 | -43 | 12 |
| Comparative Example 9 | 15 | -31 | 55 |
| Comparative Example 10 | 15 | -32 | 29 |
| Comparative Example 11 | -2 | -45 | 79 |
| Comparative Example 12 | 12 | -31 | 25 |
| Comparative Example 13 | -1 | -40 | 109 |
| Comparative Example 14 | 32 | -30 | 6 |
| Comparative Example 15 | 24 | -29 | 22 |

As a result of the experiment, all of Examples 1 to 5 exhibited a swelling value of 20 or higher, an absolute wettability value of 30 or lower, and a strength value of 20 or lower, and therefore, it was found that the swelling, wettability, and strength of the hydrogels were very excellent.

On the other hand, Comparative Examples 1 to 11, where one or more of the components constituting the first gelling agent and the second gelling agent was excluded, exhibited a swelling value lower than 20, an absolute wettability value higher than 30, or a strength value of higher than 30, and therefore, it was confirmed that at least one property of swelling, wettability, and strength of the hydrogels was poor.

In addition, Comparative Examples 12 to 15, where the first gelling agent and the second gelling agent were included outside the weight ratio range of the examples, also exhibited a swelling value lower than 20, an absolute wettability value higher than 30, or a strength value of higher than 30, and therefore, it was confirmed that at least one property of swelling, wettability, and strength of the hydrogels was poor.

### (2) Experimental Example 2: Evaluation of the feeling of use

To evaluate the feeling of use of the compositions according to Examples 1 to 5 and Comparative Examples 1 to 15, 30 evaluators in their 20s to 40s were each asked to evaluate the rolling sensation and residual sensation, and the results are shown in Table 5.

The experimental method is as follows. After the evaluators applied the samples of Examples and Comparative Examples to their skin, they evaluated the evaluation items on a five-point scale, and the scores were given in units of one point for each level, with a full score of five points. The feeling of use was evaluated by calculating the average value by adding up all the scores and then excluding the deviation.

**[Table 5]**

| Classification | Rolling sensation | Residual sensation |
|---|---|---|
| Example 1 | 5 | 4 |
| Example 2 | 4 | 5 |
| Example 3 | 5 | 5 |
| Example 4 | 4 | 4 |
| Example 5 | 4 | 3 |
| Comparative Example 1 | 2 | 3 |
| Comparative Example 2 | 1 | 2 |
| Comparative Example 3 | 2 | 3 |
| Comparative Example 4 | 1 | 2 |
| Comparative Example 5 | 2 | 3 |
| Comparative Example 6 | 1 | 1 |
| Comparative Example 7 | 1 | 1 |
| Comparative Example 8 | 2 | 3 |
| Comparative Example 9 | 1 | 1 |
| Comparative Example 10 | 3 | 3 |
| Comparative Example 11 | 1 | 1 |
| Comparative Example 12 | 1 | 2 |
| Comparative Example 13 | 1 | 2 |
| Comparative Example 14 | 2 | 2 |
| Comparative Example 15 | 2 | 2 |
| <Evaluation items> | | |
| - Rolling sensation: The more the hydrogel composition is rolled and applied, the higher the score. | | |
| - Residual sensation: The more the hydrogel composition that is rolled on the skin remains, the higher the score. | | |

As a result of the experiment, it was found that all of Examples 1 to 5 exhibited high scores for evaluation items of rolling sensation and residual sensation, indicating that they had an excellent feeling of use.

On the other hand, all of Comparative Examples 1 to 11, where one or more of the components constituting the first gelling agent and the second gelling agent was excluded, exhibited low scores for evaluation items of rolling sensation and residual sensation, confirming that they had a poor feeling of use.

In addition, all of Comparative Examples 12 to 15, where the first gelling agent and the second gelling agent were included outside the weight ratio range of the examples, also exhibited low scores for evaluation items of rolling sensation and residual sensation, confirming that they had a poor feeling of use.

Although the present invention has been described in detail with reference to the specific features, it will be apparent to those skilled in the art that this description is only for a preferred embodiment and does not limit the scope of the present invention. Thus, the substantial scope of the present invention will be defined by the appended claims and equivalents thereof.

## Claims

1. A hydrogel composition comprising:
a first gelling agent; and
a second gelling agent,
wherein the first gelling agent includes alginate, carrageenan, and gellan gum, and the second gelling agent is succinoglycan.

2. The composition of claim 1, wherein the alginate, the carrageenan, the gellan gum, and the succinoglycan are included in a weight ratio of 1:0.5 to 8:0.5 to 2:0.5 to 4.

3. The composition of claim 1, wherein the alginate is included in an amount of 0.01% to 10% by weight based on the total weight of the composition.

4. The composition of claim 1, wherein the carrageenan is included in an amount of 0.01% to 10% by weight based on the total weight of the composition.

5. The composition of claim 1, wherein the gellan gum is included in an amount of 0.01% to 10% by weight based on the total weight of the composition.

6. The composition of claim 1, wherein the succinoglycan is included in an amount of 0.01% to 10% by weight based on the total weight of the composition.

7. The composition of claim 1, wherein the first gelling agent further includes one or more selected from the group consisting of glucomannan, xanthan gum, gelatin, chitosan, collagen, hyaluronic acid, gum arabic tree (*Acacia senegal* gum), pullulan, pectin, cellulose, sclerotium gum, tara gum (*Caesalpinia spinosa* gum), agar, locust bean gum, and guar gum (*Cyamopsis tetragonoloba* gum).

8. The composition of claim 1, further comprising at least one of a curing agent and a moisturizing agent.

9. The composition of claim 8, wherein the curing agent includes at least one of a divalent metal ion, a trivalent metal ion, and a metal salt.

10. The composition of claim 9, wherein the metal ion includes one or more selected from the group consisting of calcium (Ca²⁺), magnesium (Mg²⁺), barium (Ba²⁺), manganese (Mn²⁺), nickel (Ni²⁺), copper (Cu²⁺), zinc (Zn²⁺), iron (Fe³⁺), and aluminum (Al³⁺).

11. The composition of claim 9, wherein the metal salt includes one or more selected from the group consisting of calcium chloride, calcium hydroxide, calcium nitrate, and calcium sulfide.

12. The composition of claim 8, wherein the curing agent is included in an amount of 0.01% to 5% by weight based on the total weight of the composition.

13. The composition of claim 8, wherein the moisturizing agent includes one or more selected from the group consisting of glycerin, propylene glycol, dipropylene glycol, butylene glycol, pentylene glycol, and sorbitol.

14. The composition of claim 8, wherein the moisturizing agent is included in an amount of 0.01% to 35% by weight based on the total weight of the composition.

15. A method of preparing a hydrogel composition, the method comprising:
a step of mixing a first gelling agent and a second gelling agent in purified water to obtain a mixture; and
a step of dropping the mixture into a curing agent,
wherein the first gelling agent includes alginate, carrageenan, and gellan gum, and the second gelling agent is succinoglycan.
